# EUROPEAN PATENT APPLICATION

(11) **EP 3 888 681 A2**
(43) Date of publication of application: **06.10.2021**
(21) Application number: 19889430.5
(22) Date of filing: 25.11.2019
(51) Int. Cl.: A61K 45/00, A61K 33/00, A61K 31/00, A23L 33/16, A61P 35/00, A61P 37/02, A23L 33/10, A61P 37/06

(54) **SUBSTANCE FOR TREATING AND/OR PREVENTING TUMORS, AND DESIGN METHOD AND PREPARATION METHOD THEREFOR**

(30) Priority: 28.11.2018 CN 201811437559; 25.08.2019 CN 201910787172
(71) Applicant: Dong, Futian, Suzhou, Jiangsu 215123 (CN)
(72) Inventor: Dong, Futian, Suzhou, Jiangsu 215123 (CN)
(74) Representative: Biggi, Cristina
(86) International application number: PCT/CN2019/120739
(87) International publication number: WO 2020/108456

(57) **Abstract**

Provided is a method of designing and preparing a substance for treating and/or preventing a tumor, wherein, a stable structure corresponding to a tumor-related substance is used as the substance for treating and/or preventing the tumor. The essence of disease is the imbalance of organism structural system. The imbalance of the structure of substance in the organism structure system will cause diseases related to the substance. Disclosed is a method that the organism regulates genes or the expression of genes, restores the balance of the structures of substances in the organism structural system, and treats and/or prevents a disease related to the tumor, through the self-adaptation and self-organization of the structure system of the organism after recognizing the stable structure corresponding to the substance.

## Description

### FIELD OF DISCLOSURE

The disclosure belongs to the fields of medicines, health foods, food additives and the like, and specifically relates to substances for the prevention and/or treatment of tumors and design methods and preparation methods thereof.

### BACKGROUND OF DISCLOSURE

Tumor refers to a new organism formed by the proliferation of local tissue cells under the action of various tumor-causing factors. According to the harm caused by tumor and the growth of tumor, it can be divided into benign tumor and malignant tumor. Benign tumors grow slowly and expansively, usually have a complete envelope on the surface. They cause local symptoms with less systemic symptoms. They do not infiltrate the surrounding tissues or metastasize to the whole body. Benign tumors are not easy to recur after surgical resection, and cause less harm to the body. Examples of the benign tumors includeLipoma, hemangioma, adenoma, cyst and the like. Malignant tumors grow rapidly and often infiltrate the surrounding tissues, usually have little envelope on the surface and often cause systemic metastases. Pathological examinations of malignant tumors show atypical mitosis. In addition to local symptoms, systemic symptoms of malignant tumors are obvious. Advanced patients often suffer from cachexia, high recurrence rate after surgical resection and great harm to the body. Malignant tumors include liver cancer, lung cancer, leukemia, osteosarcoma, etc. Currently, tumor is the first one on the leading causes of death and has become a major health threat.

The traditional treatments for malignant tumors are surgery, radiation, and chemotherapy. Surgery is the most important method for the treatment of malignant tumors. For early- and mid-term malignant tumors, surgery should be considered as the first choice for treatment. Radiotherapy and chemotherapy are mainly suitable for the comprehensive treatment of mid-term and advanced tumors and cancers. However, the above three conventional treatment methods have certain limitations and cause certain damage to the patient's body. In particular, radiotherapy and chemotherapy have different degrees of inhibition on the normal immune system, and their cure rates are not high.

According to a new medical model, structural information medicine, the present disclosure recognizes life from the perspective of structural systems, and restores the dynamic balance of structural systems by allowing the organism to recognize the information of specific structures and using the self-adaptation and self-organization of the organism's structural system, thereby achieving the purpose of preventing and treating diseases.

### 1. Medical Model of Traditional Chinese Medicine and Western Medicine

The existing medical system includes western medicine and traditional Chinese medicine (TCM). Western medicine is based on the microscopic local material structure, which is good at studying but short of reasoning. Traditional Chinese medicine is mainly based on the macroscopic overall functional system, and is good at reasoning while clumsy at studying. The advantages of western medicine and TCM complement each other, while the theory and research methods are opposed to each other. Western medicine mainly relies on scientific experiments, using basic research methods such as anatomical physiology, biochemistry and molecular science, and clinical trials to analyze the material nature of diseases through the theory of reductionism. Therefore, western medicine pays more attention to the diseased structural material, analyzes the changes in the specific structure, and then uses chemical drugs, surgery, radiation and other methods to directly reduce the superfluous part and supplement the insufficient part at the level of the local material structure, such as treating hyperacidity with H2 receptor antagonists, hormone replacement therapy, etc. Such therapy acts quickly and is effective in some acute diseases, surgical diseases and infectious diseases. However, western medicine lacks a comprehensive understanding of the body. Said therapy can only temporarily solve the problem of local structure. Long-term use of western medicine will have an adverse effect on other structures of the body and disrupt the balance of the body. In fact, since 2010, one third of the diseases in the world are pharmaceutical-induced diseases, which are caused by the inevitable side effects of pharmaceuticals. In addition, western medicine has little effect on chronic degenerative diseases with complex etiology affected by multiple factors, such as cardiovascular and cerebrovascular disease, multiple sclerosis, and senile dementia. It is difficult to curb the increasing morbidity and mortality by western medicine.

TCM considers life as a whole, unites people with society, psychology, and environment, takes treatment based on syndrome differentiation as the core, and reflects the objective laws of nature and people, health and disease. TCM emphasizes the relative balance, coordinated and orderly state of the body's Yin and Yang in the subjective and objective environment, that is, the balance of the functional system. When this balance is broken, the order and homeostasis of the human body is disturbed and destroyed, that is, the functional system of the human body is out of balance, and diseases will occur. TCM diagnoses the nature of the disease by inquiring a large amount of information of the functional system, and then adjusts the body's functional system through drugs, acupuncture, massage and other methods. On the whole, TCM reduces the superfluous part and supplements the insufficient part, strengthens the body resistance to eliminate pathogenic factors, and restores the body's homeostasis. TCM has a slow onset of action, but it can reduce the side effects of alternative methods or antagonistic drugs, and is quite effective in disease prevention and treatment, and health preservation and rehabilitation.

In sum, western medicine pays more attention to microscopic structure balance, while TCM pays more attention to macroscopic functional system balance, both of which aim to correct the imbalance of the organism at different levels. However, they are completely different in the epistemology and methodology of disease and health. In order for the two medical models to complement each other, we need to integrate them with a more inclusive perspective and theory, and develop more effective treatments for diseases.

### 2. Structural information medicine

### (1) Integrate the basic theories of western medicine and TCM with structural system

The Big Bang brings positive and negative charges, which interacted to form a simple structure of hydrogen. Then the interaction among the structures formed a more complex structure, and finally formed a macroscopic matter, including the entire biological world. Therefore, everything in the universe is unified to structure. The substance, energy, and information that make up everything in the universe are also unified in structure. First of all, different structures constitute different substances. Substances are specific structures and are the basis for functions. The structure and function of a living system are matched, i.e., specific structure has specific function(s), and the change of specific structure naturally affects the specific function(s) of the corresponding system. Secondly, the bond energy of the interaction among the structures is a relatively abstract structure, indicating the stability of the structure. Energy is the internal driving force for the interconnection of the structures. Finally, the relationship and order among structures belongs to information, which is the most abstract structure among the three. The information combines and controls substance and energy, reflecting the internal laws and nature of the structure. For example, the nature of biology is determined by its abstract structural genes. Therefore, structure is the unity of substance, energy and information, representing the unity of concrete structure and abstract structure.

An organism is an organic system, essentially an organic structural system dominated by abstract structures. The structural system is an organic structure with a certain function formed by the relatively stable contact, organizational order and temporal and spatial relation among several elements. Any element itself is also a structural system, a sub-system that constitutes the original system, and the sub-system must be composed of secondary sub-systems... The structural system of an organism is composed of structural sub-systems of different levels, such as the immune system and the gastrointestinal system. And the organism itself is a sub-system of a larger structural system. For example, organism is a sub-system of nature, a larger structural system. Organisms maintain their own existence and reproduction by the constant exchange of substance, energy and information with nature.

As the genetic material of organisms, DNA contains all the information involved in the life, including growth, development, aging and death. As in the biochemical individualization theory, Dr. Roger Williams considers that the difference in the biochemical composition of the human body comes from the difference in genes and gene expression, that is, the difference in the molecular spatial structure of gene expression. Nobel Laureate in Physiology Dr. Tonegawa Susumu stated that all diseases except trauma are related to genes. The causes of diseases mainly include two aspects: one is that the structure that constitutes the organism is damaged or changed by the structural environment; the other is improper gene expression. Structural imbalance is caused by the change of abstract structure, that is, the change of gene expression, such as abnormal histone acetylation and methylation, abnormal gene methylation, etc. If the specific structure has changed, as long as the genes related to the structure can be expressed normally, the organism's metabolism will gradually replace the diseased structure with the normal structure, and the organism can return to a healthy state. Therefore, the core of biological health lies in genes and the expression of genes.

Western medicine studies specific structural changes, using disease as a model, and treating diseases based on the model. Western medicine focuses on the study of local tissues and functions in structural systems, which can be detected by instruments. Western medicine studies the specific and microscopic material structure, focusing on and emphasizing the existence of specific human material structures and forms, such as anatomy, molecular biology, cytology, histology and embryology, etc. These disciplines research and observe specific structures in structural systems from different angles by experimentation. Western medicine is insufficient in abstract thinking and ignores the integrity of the organism as a structural system. Therefore, it is easy to be limited on understanding and practice. Western medicine mainly directly intervenes in specific structures, changes and affects the local structure of the organism by drugs to achieve a new balance. For example, research on genes in western medicine tends to directly change genes or their expression. TCM considers humans as a model. It studies the functions of the various organs of the organism as well as the interrelationships and effects among the functions, with holism as the leading ideology. TCM does not detect specific material structures, but diagnoses diseases through functional manifestations and external information. But the function is determined by the structure. The essence of the functional system is the structural system. The mutual promotion and restraint among the functional sub-systems are essentially the mutual promotion and mutual restriction among the structural sub-systems. TCM achieves the overall balance by regulating the order of functions, strength and weakness of the organism. Its essence is to restore the dynamic balance of the structural system. However, due to the lack of in-depth understanding of the specific organism structure, it also has great limitations. Western medicine establishes disease models from the perspective of microstructure, while TCM establishes life models from the perspective of macroscopic function. The microstructure of western medicine has found the material basis for the TCM syndromes, that is, TCM syndromes has material support of gene expression, i.e. structural support. Therefore, the essences of TCM and western medicine are both microscopic structural imbalance. In the structural system, based on the unity of the micro view and the macro view, of the entirety and the parts, and of the concrete and the abstract, the partial and micro concrete structure can be used to transform the concrete structure into abstract structural information through the identification of the concrete structure by the organism. This could stimulate self-adaptive and self-organizing functions of the organism to adjust genes or the expression of genes, and to regain the balance of the structural system.

A structural system has the basic characteristics of a system: integrity, openness, stability, and self-organization. As compared to the environment, system is the unity of closeness and openness. The interaction among the structures in the structural system is based on the interaction among the structures and the environment, and depends on the external environment of the structures. Meanwhile, the evolution of the structural system depends on the external structure. Proper change and adjustment of gene expression along with changes in external structure is the basic ability of organisms to adapt to the environment, keep survival, and maintain their own health, without which an organism will generally be eliminated. The structural system of the human body has this powerful self-care function. Structural information medicine is to mobilize and regulate the relatively complex, highly ordered, and self-regulated mechanism constituted by the sub-systems of the structural system at different levels including genes, molecules, cells, tissues and organs through the structural information of the specific structure, so as to restore the structural balance of the organism and achieve the purpose of preventing and curing diseases. Structural information medicine integrates western medicine's theory of specific structure and function as the research object with TCM's theory of balance restoration by regulating the organism itself.

### (2) Problems solved by structural information medicine

Living organisms cannot exist independently from the environment. The external structural environment interacts with the structural system of the organism through structures, namely matter, energy and information. Each individual is a unique structural system resulting from the interaction between genes and environment. Structural information medicine emphasizes the biological internal structural system and its dynamic balance with the external structural environment. It is this dynamic balance that interprets the process of life. In addition to restoring the dynamic balance of the structural system of the organism and increasing the body resistibility, structural information medicine focuses on symbiosis and co-progress. Symbiosis means that the organism no longer blindly resists and defends the external structure, but constantly internalizes the external structure information, and realizes the balance between the structural system of the organism and the external structure through the self-adaptation and self-organization of the organism. This process may turn the enemy into a friend, even let the enemy serve us. Co-progress means that the structural system of an organism obtains the structural characteristics of other organisms that are conducive to the health, survival and reproduction of the organism through structural information. The present disclosure is based on the established theoretical system of structural information medicine. By using the stable structure corresponding to a substance, the disclosure provides a method that regulates genes or the expression of genes of an organism through the self-adaptation and self-organization of the structural system of the organism, and treats a disease related to the substance.

### SUMMARY OF DISCLOSURE

The purpose of the present disclosure is to provide a method for designing a substance for treating and/or preventing tumors in response to the above-mentioned defects of the prior art.

Another purpose of the present disclosure is to provide a substance designed according to such method.

Another purpose of the present disclosure is to provide a method for preparing the substance and use thereof.

Organism structural system is an organic structural system with uniform stability and adaptability. It can adapt to the environment, maintain the dynamic balance between itself and the external environment, and then achieve better survival and reproduction of itself. The adaptability of the organism structural system is based on the stability of the biological structure. A stable structure is the dominant structure that plays a key role.

Organism structural system is an organic giant system formed by spiral development of core structure composed of concrete structures and abstract structures. The core structure of the organism structural system is the unified complex of the concrete structures and the abstract structures in the nucleic acid-protein structural system. As a genetic information carrier and expression system, the core structure plays a dominant role in the function of the organism structural system, while the stable structure plays a dominant role in the core structure. At the atomic level, the abstract structure DNA and the concrete structure protein in the core structure are both biological macromolecules based on the carbon element. There are 4 electrons in the outermost layer of a carbon atom. Such structure is not easy to lose or get electrons, and is conducive to form 4 covalent bonds. The covalent bond has high stability and is the strongest force in the organic molecules. The single covalent bond mainly formed by carbon atoms has the bond energy about 300-500 kJ/mol, with the thermal degradation temperature of 500-800°C.

The primary structure of DNA determines its double helix secondary structure through base hydrogen bonding and base stacking force. Therefore, the stable primary structure determines its adaptive secondary structure. The core of genetic information lies on the stable structure. DNA transfers genetic information to RNA through transcription, and finally RNA expresses genetic information into protein through translation. This is the law followed by all cellular organisms. The genetic central dogma clarifies the information transmission path of genetic information from DNA to RNA and then to protein. From the base sequence in the primary structure of DNA to the base sequence in the primary structure of RNA, then to the amino acid sequence in the primary structure of protein, the genetic information carrier maps the information one-to-one to the functional information carrier amino acids through base complementary pairing and triplet codons. Therefore, this information transmission path is based on the stable structure of these biological macromolecules, and starts from the stable abstract structure to the stable concrete structure.

The covalent backbone in the primary structure of a protein contains hundreds of single bonds, which can rotate freely. However, the rigid planar nature of the peptide bonds in the primary structure and the number and position of hydrophilic and hydrophobic amino acids and acid-base amino acids restrict the freedom of rotation of each single bond, and finally form a most thermodynamically stable three-dimensional structure. Therefore, the component that determines the final structure of the protein is the amino acid sequence, which is formed very early. The stable structure that dominates the protein conformation forms a framework. During the folding of the polypeptide, the protein is assembled into the final form according to this framework. The amino acid sequence of a protein contains all the information that determines its three-dimensional structure, and the structure of the protein determines the function of the protein. Therefore, as the case for the genetic information, the nature of functional information is that the stable structure determines the adaptive structure.

From the perspective of the system, the stability of an organism structural system is the self-stabilizing ability of the organic structural system formed by structural interaction. Each structure in the structural system has its promoting structure and its inhibiting structure, which form a dynamic balance by interaction, that is, they cannot be insufficient or too much. Each structure can adjust itself within a certain range according to environmental changes, thereby maintaining and restoring the original orderly state and maintaining its own orderliness in an unbalanced state. During such process, the interaction of the stable structures plays a dominant role. For example, during the recognition of structural information, the innate immune system in animals and plants can recognize the structural characteristics of pathogens through pattern recognition receptors (PRR). These characteristics are the stable molecular structures of different types of pathogens that are not easy to mutate (such as all bacteria have a cell wall composed of LPS). In the case of food allergy, even if the food containing an allergen is treated with high temperature, it will still cause allergy, in which the stable structure of the allergen can be recognized by the immune system. As another example, neurotransmitter opioid peptides of different stereo conformations have different selectivity for cell membrane receptors. Endorphin-1 is a known biologically active peptide with the highest affinity and selectivity to µ receptors. The adaptive structure is the molecular basis for the binding of signal molecules and receptors. As for the amino acids (i.e. the stable structures), the difference between endomorphin-1 and other opioid peptides lies mainly in three amino acids. The change of amino acid residues forms an appropriate spatial layout, changes the three-dimensional spatial conformation, and plays a role of correct positioning for the binding of such transmitters and receptors. The interaction among the stable structures can cause significant changes in the adaptive structures and play a huge role in the information recognition. During information transmission, the receptors on the cell membrane bring the information to the nucleus, which mainly depends on the modification of the protein. The basis of protein modification is the chemical reaction activity of amino acid residues in the primary structure. Under the action of enzymes, the stable structure of the protein can be adjusted to adapt the high-level structure of the protein to the functional requirements under specific time and space conditions. Such process is reversible and provides multi-functions, such as phosphorylation and de-phosphorylation of proteins. For the information transmitted to the nucleus, the cell will eventually make corresponding feedback through changes in gene expression, and further transfers instructions through signal pathways. Therefore, the interaction or the adjustment among the stable structures determines the change of the adaptive structures. And the adaptive structures can also counteract the stable structures.

Among the organism structural systems, no structure is "isolated". The interaction, mutual restriction and interdependence among the structures and the sub-structural systems constitute the biological organic structural system. All organisms are made up of cells. Cells are the sub-structural system of this large structural system of the organism. The organism is composed of hundreds, tens of thousands, or even hundreds of millions of cells that perform various specific functions, which constitutes an orderly and controllable cell society. This kind of social maintenance depends not only on the material metabolism and energy metabolism of the cells, but also depends on the communication and signal regulation among cells, so as to coordinate the behavior of the cells, such as cell growth, division, differentiation, apoptosis and various other physiological functions, and to realize the complete life activity process of multicellular organisms. The essence of intercellular communication and signal regulation also belongs to interactions among structures to determine the behavior and fate of cells, including structural and functional differentiation, location, and life and death decisions. The morphological structure, life activities, and position of cells in the body are all regulated and controlled by the body, local tissues, surrounding cells, and extracellular signal molecules. For example, nerve cells, immune cells and endocrine cells participate and maintain steady-state balance of the body through social connections. The interaction among structures in the organism structural system allows the cell to have the ability to distinguish itself from the dissident, and to distinguish the same species from heterogeneous cells. The recognition and differentiation among cells, as well as the recognition of autologous and allogeneic substances is called cell recognition. Cells adhere to each other or establish a stable connection with a certain morphological structure on the basis of recognition, thereby influencing and interacting with each other. Many important life activities are related to the recognition ability of cells. Cell recognition is also a very important segment in the process of cell development and differentiation. Cells form different types of tissues through recognition and adhesion. Immunity is a kind of recognition of cells. White blood cells in the blood can recognize invading bacteria and eliminate them, but never affect normal autologous cells in the blood. This is the recognition of cells for different species. In allogeneic tissue transplantation, the body will reject the transplanted allogeneic tissue. The rejection is caused by cell recognition for cells from different sources. In addition, many physiological and pathological processes such as blood coagulation, inflammatory reaction, thrombosis, infection of pathogenic microorganisms and even tumor cell metastasis are related to the complex mutual recognition and adhesion of homogeneous or heterogeneous cells and the induced effects thereof. Structural recognition is based on the interaction among structures, and the interaction among structures is the basis of the self-adaptive, self-organizing organic structural system formed by living things.

The openness of the structural system is not only the openness of specific structures, but also the openness of relationships. The self-adaptive and self-organizing ability of the structural system is mainly reflected by the stability of the system under external environmental stimuli. The structural system self-adjusts and self-organizes through the transmission and processing of information, constantly overcomes the uncertainty of the structural system, and maintains a dynamic balance. The exchange between the structural system and the environment is not only the exchange of substance, but also the exchange of energy or information. To maintain the stability of the internal environment in a constantly changing environment, organisms need the ability of cells to sense and respond to these changes. The ability of cells to receive and transmit information and execute instructions is the basis for organisms to respond to changes in internal and external environments. Proteins are the performers of various physiological activities in cells (such as catalyzing chemical reactions and regulating gene expression), and changes in proteins will also change the functional state. Therefore, proteins are used by organisms to build a signaling system. Because the shape of a protein molecule can be changed, the functions closely related to such shape will change accordingly, and a change in the state of one protein will change the state of the downstream molecule through the interaction with the downstream protein molecule, and the downstream molecule can change the state of further downstream molecules. Such successive changes in the state of the cells are the way of information transmission in the cell. Moreover, a protein is a molecule with biological functions that makes the final response to a signal after a state change, and becomes an effector molecule. The effector molecules at the end of the information transmission chain are mostly transcription factors, which respond to signals by regulating the expression of genes.

For example, any foreign structure will be recognized and evaluated by the immune system when entering the human body. When bacteria or viruses enter the human body, immune cells interact with foreign structures to recognize their structural information. According to the different structural information, immune cells secrete different cytokines to transmit the information to B lymphocytes, and regulate gene expression of B cells to synthesize IgG antibody. The antibody binds to the antigen structure to form a complex, which is eventually cleared by macrophages to resist the foreign invasion. When parasitic pathogens enter the human body, immune cells interact with foreign structures to recognize their structural information and activate B cells, and then regulate gene expression to synthesize IgE antibody. These antibodies bind to mast cells and basophils to release toxic granule protein to kill parasites.

In such immune response process, the recognized structure determines the specific feedback effect of the organism. Based on the interaction between the organism and the structure, the specific structure is transformed into the abstract structure, i.e., structural information. According to the identification of the structural information, the organism mobilizes the synergy of multiple cells in the immune system, regulates gene expression, and releases different active substances to act with the foreign substance, and then achieves a new stable and harmonious structural system. At the same time, the immune system interacts with other systems to exchange substances, energy, and information to promote the coordination of different subsystems in the structural system. It can be seen that there is a balance mechanism in the structural system of the organism, including structural information recognition and self-regulation, restoration of the balance of the structural system, and even establishment of a new structure with the external structure.

Disease is an abnormal life activity process that occurs due to a disorder of homeostasis under certain factors, which triggers a series of changes in metabolism, function and structure. The disease manifests itself as abnormal symptoms, signs, and behavior. The disease is an abnormal life activity process that occurs due to the disorder of homeostasis after the body is damaged by the cause of the disease under certain conditions. From the structural point of view, biological diseases are caused by the imbalance of the organism structural system, including: (1) the structural imbalance within the organism structural system (2) the structural imbalance between the organism structural system and the external structure, that is, the organism structural system lacks a substance that inhibits the external structure. Therefore, a substance related to a disease include: a substance that causes the disease, a substance in an organism related to the disease, and a substance that inhibits the structure that causes the disease. The substance that inhibits the structure that causes the disease is a substance that can establish a structural balance with the structure that causes the disease. Disease is an extremely complex process. In many cases, from health to disease is a process from quantitative change to qualitative change. Some diseases involve the structural imbalance of multiple systems in the organism structural system. The "zheng" of TCM reflects the essence of the disease, and treatment requires differentiation of the "zheng". The "zheng" is neither a disease nor a symptom of western medicine, nor a generalization of a single factor or linear causality of the disease. Based on the unique theories and methods of TCM, "zheng" is the understanding and generalization of the body's response state by connecting various physiological and pathological factors such as cause, location, pathogenesis, pathological nature of the disease, and interfering between healthy Qi and pathogenic Qi. It focuses on the unity of opposites for contradiction between healthy Qi and pathogenic Qi. Therefore, both "strengthening healthy Qi to dispel pathogenic Qi" and "dispelling pathogenic Qi to hold healthy Qi" focus on healthy Qi and pathogenic Qi. The basic principle of TCM is to adjust Yin and Yang of the human body, restore its relative balance and coordinate and orderly stable state of healthy Qi and pathogenic Qi.

Western medicine studies health and disease by investigating the relationship between structure and function from a concrete and micro perspective. Western medicine studies health and disease by investigating the multi-dimensional and two-way adjustment relationship of restriction and generation between the human body's functional systems from an abstract and macro perspective. Structurally, the essence of organisms is a dynamic balance, coordinated and orderly adaptive, self-organizing organic structural system formed by the interaction among body structures. Structural information medicine integrates the basic theories of TCM and Western medicine with the concept of structure, recognizes life from the perspective of structural systems, and restores the dynamic balance of structural systems by allowing the body to recognize the information of specific structures and using the self-adaptation and self-organization of the body's structural system, thereby achieving the purpose of preventing and treating diseases.

Different lesions of the same organ can lead to different diseases; the symptoms of the same disease in different individuals are not exactly the same; the same disease may involve the lesions of multiple different organs or tissues in the organism structural system; and the diseased structure of the same disease in different individuals is not exactly the same. Therefore, the present disclosure treat and/or prevent diseases from the perspective of structural information by combining the specific structure of Western medicine research and the macro theory established by TCM. For a disease, only the disease-related substances and the specific diseased cells, tissues or organs containing the disease-related substances need to be known. It does not need to explore the mechanism through which the disease is caused, the specific symptoms of the disease, or the system where the disease-related substances are located. The disclosure is useful especially when the specific diseased structure cannot be determined, or it is not easy to separate the diseased structure. Systemically, humans are the same according to either Western medicine or TCM, even humans and swine, cattle, sheep, primates are basically the same. Therefore, it is more versatile to use stable structures corresponding to cells, tissues or organs that contain such diseased structure (that is, the unbalanced structure) for disease treatment and/or prevention, which is easier to obtain and prepare. Therefore, the method for designing substances for the treatment and/or prevention of diseases disclosed in the present disclosure applies a stable structure corresponding to the disease-related substance as an external structure to the structural system of the organism. The organism structural system can make the organism establish a balance with the structure of the external substance or restore the inherent structural imbalance of the organism structural system according to the self-adaptation and self-organization of structures, thereby eliminating, alleviating, or preventing the occurrence of diseases. For example, asthma or a disease related to lung (such as allergic rhinitis that interacts with the structure of the lung) can be treated and/or prevented by a stable structure corresponding to swine lungs. The method of designing substances for the treatment and/or prevention of diseases in the present disclosure mainly includes strengthening healthy Qi with pathogenic Qi and strengthening the healthy Qi with healthy Qi:

The imbalance of the organism structural system includes the imbalance between the organism structural system and the structure other than itself. In the present disclosure, the organism structural system itself is called "healthy Qi", and the external structure that causes the imbalance of the organism structural system is called "external pathogenic Qi". The "external pathogenic Qi" is relative to organisms, including substances that are harmful to healthy organisms (such as bacteria and viruses that cause diseases), and substances that are harmless to healthy organisms, while for a certain organism, these substances do not cause diseases when the organism does not contact the substances but will cause diseases after the contact. For example, a certain substance is harmless or even beneficial to healthy organisms, which means that there is a balance between the structure of the substance and the structure of the organisms. But if the structure of the organisms is out of balance, the balance between the substance and the organism structure is broken. Different imbalances can cause different diseases, such as allergies, intolerances, including milk allergy and milk intolerance. According to the self-adaptive and self-organizing function of the organism structural system to maintain structural stability, under the action of the external structure, a structure that inhibits or promotes the external structure will be generated, thereby establishing the balance between the organism structural system and the external structure, i.e., one kind of strengthening "healthy Qi" with "external pathogenic Qi".

The pathological changes in the internal structure of the organism structural system are often the imbalance of structural interactions. For example, for a certain structure in the organism structural system, lacking of the expression of genes that restrict the structure or overexpression of genes that promote the structure will break the balance between structures. For example, tumor suppressor genes and proto-oncogenes restrict each other and maintain the relative stability of positive and negative regulatory signals. When such genes are mutated, deleted or inactivated, the balance of interactions among structures is broken, which can cause malignant transformation of cells and lead to tumors. In the present disclosure, the unbalanced structure in the organism structural system is called "internal pathogenic Qi", and the organism structural system is called "healthy Qi". The stable structure corresponding to the substance causing unbalanced internal structure of the organism as disclosed in the present disclosure acts as an external structure on the organism structural system. Due to the self-adaptive, self-organizing and self-stabilizing functions of the organism structural system, the organism structural system regulates the expression of genes in structures that interacts with the external structure according to the structural information in the external structure, so as to achieve a balance with the external structure, restore the balance of the internal unbalanced structure in the organism structural system, and cure the disease, i.e., one kind of strengthening "healthy Qi" with "internal pathogenic Qi".

Every normal structure in the organism structural system has a structure that interacts with the normal structure, that is, a balanced structure. The present disclosure refers to the balanced structure and the organism structural system as "healthy Qi". When a stable structure corresponding to the normal substances without disease in the system is used as an external structure to act on the organism structural system, due to the self-adaptive, self-organizing, and self-stabilizing functions of the organism structural system, the organism structural system regulates the expression of genes interacting with the external structure to standard levels according to the structural information of each structure in the external structure, and the unbalanced structure will naturally be back to balance. Tumors are gene mutations or abnormal gene expression, which are essentially imbalances of the structural system established by abstract and concrete structures. For example, when the nucleus of a cancer cell is transplanted into a normal fertilized egg, because the genes are in a normal and balanced structural environment, the expression of the genes can return to normal, and the egg can develop into a healthy organism. Therefore, by the treatment of normal external structure, the expression of cancer genes can be restored to normal, so that tumor cells can develop into normal cells. The present disclosure discloses that a structure corresponding to the substance without a tumor can be used as a substance to treat or prevent diseases caused by the lesions of the same or similar substance as the substance without a tumor, i.e., one kind of strengthening "healthy Qi" with "healthy Qi". For example, lung cancer can be treated and/or prevented by a stable structure corresponding to a healthy swine lung.

The balance of the organism structural system is dynamic, especially the balance between the organism structural system and the structure of the environment in which it is located. For example, if a substance that can cause disease does not cause the disease in a certain organism, the structural system of the certain organism can reach a balance with the structure of the disease-causing substance during interaction. Therefore, the structure of the certain organism and the disease-related structure have structures that interact with the structure of the disease-causing substance without causing the organism to produce the disease. These structures are not isolated in the organism structural system. There are structures that interact with them and thus form a balance. When a stable structure corresponding to the disease-related substance in the certain organism is used as an external structure to treat the organism structural system in need thereof, due to the self-adaptive, self-organizing and self-organized structure of the organism structural system, the organism structural system regulates the genes or the expression of genes in structures that interacts with the external structure according to the structural information of each structure in the external structure, so that the organism structural system can be balanced with the disease-causing substance, i.e., one kind of strengthening "healthy Qi" with "healthy Qi".

### Features of the disclosure:

The present disclosure is based on the established theoretical system of structural information medicine. The present disclosure provides a method comprising applying a stable structure corresponding to a substance to the organism structural system, regulating genes or the expression of genes of the organism through the self-adaptation and self-organization of the organism structural system, and thus treating a disease related to the substance.

In view of this, the present disclosure provides a substance for treating and/or preventing tumors, a design method and a preparation method thereof, including the following embodiments:
A method of designing a substance for treating and/or preventing a tumor, wherein, a stable structure corresponding to a tumor-related substance is used as the substance for treating and/or preventing the tumor.

Preferably, the stable structure corresponding to the tumor-related substance is a structure of the substance in solid form under high temperature over 500 °C.

Preferably, the stable structure corresponding to the tumor-related substance is selected from one or more stable structure of the group consisting of:
(1) a stable structure corresponding to a substance that causes a tumor lesion in an organism or the corresponding substance thereof that does not cause a tumor lesion;
(2) a stable structure corresponding to the cell, tissue or organ where the tumor occurs;
(3) a stable structure corresponding to the healthy cell, tissue or organ corresponding to the primary site of the tumor;
(4) a stable structure corresponding to a substance containing the substance in any of the above (1) to (3).

A substance for treating and/or preventing the tumor designed according to the method described above.

A method of preparing the substance for treating and/or preventing a tumor described above:
the stable structure corresponding to the tumor-related substance is prepared by high-temperature carbonization.

Use of the substance for treating and/or preventing a tumor described above in the manufacture of a medicament, health product, food, food additive.

A method of designing a substance for treating and/or preventing an epithelial tissue tumor, wherein, a stable structure corresponding to an epithelial-tissue-tumor-related substance is used as the substance for treating and/or preventing the epithelial tissue tumor.

Preferably, the stable structure corresponding to the substance is a structure of the substance in solid form under high temperature over 500°C.

Preferably, the stable structure corresponding to the epithelial-tissue-tumor-related substance is selected from one or more stable structure of the group consisting of:
(1) a stable structure corresponding to a substance that causes an epithelial tissue tumor lesion in an organism or the corresponding substance thereof that does not cause an epithelial tissue tumor lesion;
(2) a stable structure corresponding to the cells, tissues or organs where the epithelial tissue tumor occurs;
(3) a stable structure corresponding to the healthy cell, tissue or organ corresponding to the primary site of the epithelial tissue tumor;
(4) a stable structure corresponding to a substance containing the substance in any of the above (1) to (3).

The epithelial tissue includes: skin, digestive organs, respiratory tract, breast, urinary organs, reproductive organs, lymphatic vessels, and endocrine organs.

A substance for treating and/or preventing the epithelial tissue tumor designed according to the method described above.

A method of preparing the substance for treating and/or preventing an epithelial tissue tumor described above:
the stable structure corresponding to the epithelial-tissue-tumor-related substance is prepared by high-temperature carbonization.

Use of the substance for treating and/or preventing an epithelial tissue tumor described above in the manufacture of a medicament, health product, food, food additive.

A method of designing a substance for treating and/or preventing a skin tumor, wherein, a stable structure corresponding to a skin-tumor-related substance is used as the substance for treating and/or preventing the skin tumor.

Preferably, the stable structure corresponding to the substance is a structure of the substance in solid form under high temperature over 500°C.

Preferably, the stable structure corresponding to the skin tumor-related substance is selected from one or more stable structure of the group consisting of:
(1) a stable structure corresponding to a substance that causes a skin tumor lesion in an organism or the corresponding substance thereof that does not cause a skin tumor lesion;
(2) a stable structure corresponding to the cells, tissues or organs where the skin tumor occurs;
(3) a stable structure corresponding to the healthy cell, tissue or organ corresponding to the primary site of the skin tumor;
(4) a stable structure corresponding to a substance containing the substance in any of the above (1) to (3).

A substance for treating and/or preventing the skin tumor designed according to the method described above.

A method of preparing the substance for treating and/or preventing a skin tumor described above:
the stable structure corresponding to the skin-tumor-related substance is prepared by high-temperature carbonization.

Use of the substance for treating and/or preventing a skin tumor described above in the manufacture of a medicament, health product, food, food additive.

A method of designing a substance for treating and/or preventing a gastric tumor, wherein, a stable structure corresponding to a gastric-tumor-related substance is used as the substance for treating and/or preventing the gastric tumor.

Preferably, the stable structure corresponding to the substance is a structure of the substance in solid form under high temperature over 500 °C.

Preferably, the stable structure corresponding to the gastric-tumor-related substance is selected from one or more stable structure of the group consisting of:
(1) a stable structure corresponding to a substance that causes a gastric tumor lesion in an organism or the corresponding substance thereof that does not cause a gastric tumor lesion;
(2) a stable structure corresponding to the cells, tissues or organs where the gastric tumor occurs;
(3) a stable structure corresponding to the healthy cell, tissue or organ corresponding to the primary site of the gastric tumor;
(4) a stable structure corresponding to a substance containing the substance in any of the above (1) to (3).

The gastric tumor mainly occurs in the stomach, and preferably a healthy stomach of the similar specie is used as a disease-related substance in the present disclosure.

A substance for treating and/or preventing the gastric tumor designed according to the method described above.

A method of preparing the substance for treating and/or preventing a gastric tumor described above:
the stable structure corresponding to the gastric-tumor-related substance is prepared by high-temperature carbonization.

Use of the substance for treating and/or preventing a gastric tumor described above in the manufacture of a medicament, health product, food, food additive.

A method of designing a substance for treating and/or preventing an esophageal tumor, wherein, a stable structure corresponding to an esophageal-tumor-related substance is used as the substance for treating and/or preventing the esophageal tumor.

Preferably, the stable structure corresponding to the substance is a structure of the substance in solid form under high temperature over 500 °C.

Preferably, the stable structure corresponding to the esophageal-tumor-related substance is selected from one or more stable structure of the group consisting of:
(1) a stable structure corresponding to a substance that causes an esophageal tumor lesion in an organism or the corresponding substance thereof that does not cause an esophageal tumor lesion;
(2) a stable structure corresponding to the cells, tissues or organs where the esophageal tumor occurs;
(3) a stable structure corresponding to the healthy cell, tissue or organ corresponding to the primary site of the esophageal tumor;
(4) a stable structure corresponding to a substance containing the substance in any of the above (1) to (3).

A substance for treating and/or preventing the esophageal tumor designed according to the method described above.

A method of preparing the substance for treating and/or preventing an esophageal tumor described above:
the stable structure corresponding to the esophageal tumor-related substance is prepared by high-temperature carbonization.

Use of the substance for treating and/or preventing an esophageal tumor described above in the manufacture of a medicament, health product, food, food additive.

A method of designing a substance for treating and/or preventing a colorectal tumor, wherein, a stable structure corresponding to a colorectal-tumor-related substance is used as the substance for treating and/or preventing the colorectal tumor.

Preferably, the stable structure corresponding to the substance is a structure of the substance in solid form under high temperature over 500°C.

Preferably, the stable structure corresponding to the colorectal-tumor-related substance is selected from one or more stable structure of the group consisting of:
(1) a stable structure corresponding to a substance that causes a colorectal tumor lesion in an organism or the corresponding substance thereof that does not cause a colorectal tumor lesion;
(2) a stable structure corresponding to the cells, tissues or organs where the colorectal tumor occurs;
(3) a stable structure corresponding to the healthy cell, tissue or organ corresponding to the primary site of the colorectal tumor;
(4) a stable structure corresponding to a substance containing the substance in any of the above (1) to (3).

A substance for treating and/or preventing the colorectal tumor designed according to the method described above.

A method of preparing the substance for treating and/or preventing a colorectal tumor described above:
the stable structure corresponding to the colorectal-tumor-related substance is prepared by high-temperature carbonization.

Use of the substance for treating and/or preventing a colorectal tumor described above in the manufacture of a medicament, health product, food, food additive.

A method of designing a substance for treating and/or preventing a liver tumor, wherein, a stable structure corresponding to a liver-tumor-related substance is used as the substance for treating and/or preventing the liver tumor.

Preferably, the stable structure corresponding to the substance is a structure of the substance in solid form under high temperature over 500°C.

Preferably, the stable structure corresponding to the liver tumor-related substance is selected from one or more stable structure of the group consisting of:
(1) a stable structure corresponding to a substance that causes a liver tumor lesion in an organism or the corresponding substance thereof that does not cause a liver tumor lesion;
(2) a stable structure corresponding to the cells, tissues or organs where the liver tumor occurs;
(3) a stable structure corresponding to the healthy cell, tissue or organ corresponding to the primary site of the liver tumor;
(4) a stable structure corresponding to a substance containing the substance in any of the above (1) to (3).

A substance for treating and/or preventing the liver tumor designed according to the method described above.

A method of preparing the substance for treating and/or preventing a liver tumor described above:
the stable structure corresponding to the liver-tumor-related substance is prepared by high-temperature carbonization.

Use of the substance for treating and/or preventing a liver tumor described above in the manufacture of a medicament, health product, food, food additive.

A method of designing a substance for treating and/or preventing a lung tumor, wherein, a stable structure corresponding to a lung-tumor-related substance is used as the substance for treating and/or preventing the lung tumor.

Preferably, the stable structure corresponding to the substance is a structure of the substance in solid form under high temperature over 500°C.

Preferably, the stable structure corresponding to the lung-tumor-related substance is selected from one or more stable structure of the group consisting of:
(1) a stable structure corresponding to a substance that causes a lung tumor lesion in an organism or the corresponding substance thereof that does not cause a lung tumor lesion;
(2) a stable structure corresponding to the cells, tissues or organs where the lung tumor occurs;
(3) a stable structure corresponding to the healthy cell, tissue or organ corresponding to the primary site of the lung tumor;
(4) a stable structure corresponding to a substance containing the substance in any of the above (1) to (3).

A substance for treating and/or preventing the lung tumor designed according to the method described above.

A method of preparing the substance for treating and/or preventing a lung tumor described above:
the stable structure corresponding to the lung-tumor-related substance is prepared by high-temperature carbonization.

Use of the substance for treating and/or preventing a lung tumor described above in the manufacture of a medicament, health product, food, food additive.

A method of designing a substance for treating and/or preventing a nasopharyngeal tumor, wherein, a stable structure corresponding to a nasopharyngeal-tumor-related substance is used as the substance for treating and/or preventing the nasopharyngeal tumor.

Preferably, the stable structure corresponding to the substance is a structure of the substance in solid form under high temperature over 500°C.

Preferably, the stable structure corresponding to the nasopharyngeal-tumor-related substance is selected from one or more stable structure of the group consisting of:
(1) a stable structure corresponding to a substance that causes a nasopharyngeal tumor lesion in an organism or the corresponding substance thereof that does not cause a nasopharyngeal tumor lesion;
(2) a stable structure corresponding to the cells, tissues or organs where the nasopharyngeal tumor occurs;
(3) a stable structure corresponding to the healthy cell, tissue or organ corresponding to the primary site of the nasopharyngeal tumor;
(4) a stable structure corresponding to a substance containing the substance in any of the above (1) to (3).

A substance for treating and/or preventing the nasopharyngeal tumor designed according to the method described above.

A method of preparing the substance for treating and/or preventing a nasopharyngeal tumor described above:
the stable structure corresponding to the nasopharyngeal-tumor-related substance is prepared by high-temperature carbonization.

Use of the substance for treating and/or preventing a nasopharyngeal tumor described above in the manufacture of a medicament, health product, food, food additive.

A method of designing a substance for treating and/or preventing a breast tumor, wherein, a stable structure corresponding to a breast-tumor-related substance is used as the substance for treating and/or preventing the breast tumor.

Preferably, the stable structure corresponding to the substance is a structure of the substance in solid form under high temperature over 500°C.

Preferably, the stable structure corresponding to the breast-tumor-related substance is selected from one or more stable structure of the group consisting of:
(1) a stable structure corresponding to a substance that causes a breast tumor lesion in an organism or the corresponding substance thereof that does not cause a breast tumor lesion;
(2) a stable structure corresponding to the cells, tissues or organs where the breast tumor occurs;
(3) a stable structure corresponding to the healthy cell, tissue or organ corresponding to the primary site of the breast tumor;
(4) a stable structure corresponding to a substance containing the substance in any of the above (1) to (3).

A substance for treating and/or preventing the breast tumor designed according to the method described above.

A method of preparing the substance for treating and/or preventing a breast tumor described above:
the stable structure corresponding to the breast-tumor-related substance is prepared by high-temperature carbonization.

Use of the substance for treating and/or preventing a breast tumor described above in the manufacture of a medicament, health product, food, food additive.

A method of designing a substance for treating and/or preventing a bladder tumor, wherein, a stable structure corresponding to a bladder-tumor-related substance is used as the substance for treating and/or preventing the bladder tumor.

Preferably, the stable structure corresponding to the substance is a structure of the substance in solid form under high temperature over 500°C .

Preferably, the stable structure corresponding to the bladder-tumor-related substance is selected from one or more stable structure of the group consisting of:
(1) a stable structure corresponding to a substance that causes a bladder tumor lesion in an organism or the corresponding substance thereof that does not cause a bladder tumor lesion;
(2) a stable structure corresponding to the cells, tissues or organs where the bladder tumor occurs;
(3) a stable structure corresponding to the healthy cell, tissue or organ corresponding to the primary site of the bladder tumor;
(4) a stable structure corresponding to a substance containing the substance in any of the above (1) to (3).

A substance for treating and/or preventing the bladder tumor designed according to the method described above.

A method of preparing the substance for treating and/or preventing a bladder tumor described above:
the stable structure corresponding to the bladder-tumor-related substance is prepared by high-temperature carbonization.

Use of the substance for treating and/or preventing a bladder tumor described above in the manufacture of a medicament, health product, food, food additive.

A method of designing a substance for treating and/or preventing a renal pelvic tumor, wherein, a stable structure corresponding to a renal-pelvic-tumor-related substance is used as the substance for treating and/or preventing the renal pelvic tumor.

Preferably, the stable structure corresponding to the substance is a structure of the substance in solid form under high temperature over 500°C .

Preferably, the stable structure corresponding to the renal-pelvic-tumor-related substance is selected from one or more stable structure of the group consisting of:
(1) a stable structure corresponding to a substance that causes a renal pelvic tumor lesion in an organism or the corresponding substance thereof that does not cause a renal pelvic tumor lesion;
(2) a stable structure corresponding to the cells, tissues or organs where the renal pelvic tumor occurs;
(3) a stable structure corresponding to the healthy cell, tissue or organ corresponding to the primary site of the renal pelvic tumor;
(4) a stable structure corresponding to a substance containing the substance in any of the above (1) to (3).

A substance for treating and/or preventing the renal pelvic tumor designed according to the method described above.

A method of preparing the substance for treating and/or preventing a renal pelvic tumor described above:
the stable structure corresponding to the renal-pelvic-tumor-related substance is prepared by high-temperature carbonization.

Use of the substance for treating and/or preventing a renal pelvic tumor described above in the manufacture of a medicament, health product, food, food additive.

A method of designing a substance for treating and/or preventing a cervical tumor, wherein, a stable structure corresponding to a cervical-tumor-related substance is used as the substance for treating and/or preventing the cervical tumor.

Preferably, the stable structure corresponding to the substance is a structure of the substance in solid form under high temperature over 500°C.

Preferably, the stable structure corresponding to the cervical-tumor-related substance is selected from one or more stable structure of the group consisting of:
(1) a stable structure corresponding to a substance that causes a cervical tumor lesion in an organism or the corresponding substance thereof that does not cause a cervical tumor lesion;
(2) a stable structure corresponding to the cells, tissues or organs where the cervical tumor occurs;
(3) a stable structure corresponding to the healthy cell, tissue or organ corresponding to the primary site of the cervical tumor;
(4) a stable structure corresponding to a substance containing the substance in any of the above (1) to (3).

A substance for treating and/or preventing the cervical tumor designed according to the method described above.

A method of preparing the substance for treating and/or preventing a cervical tumor described above:
the stable structure corresponding to the cervical-tumor-related substance is prepared by high-temperature carbonization.

Use of the substance for treating and/or preventing a cervical tumor described above in the manufacture of a medicament, health product, food, food additive.

A method of designing a substance for treating and/or preventing a uterine tumor, wherein, a stable structure corresponding to a uterine-tumor-related substance is used as the substance for treating and/or preventing the uterine tumor.

Preferably, the stable structure corresponding to the substance is a structure of the substance in solid form under high temperature over 500°C.

Preferably, the stable structure corresponding to the uterine-tumor-related substance is selected from one or more stable structure of the group consisting of:
(1) a stable structure corresponding to a substance that causes a uterine tumor lesion in an organism or the corresponding substance thereof that does not cause a uterine tumor lesion;
(2) a stable structure corresponding to the cells, tissues or organs where the uterine tumor occurs;
(3) a stable structure corresponding to the healthy cell, tissue or organ corresponding to the primary site of the uterine tumor;
(4) a stable structure corresponding to a substance containing the substance in any of the above (1) to (3).

A substance for treating and/or preventing the uterine tumor designed according to the method described above.

A method of preparing the substance for treating and/or preventing a uterine tumor described above:
the stable structure corresponding to the uterine-tumor-related substance is prepared by high-temperature carbonization.

Use of the substance for treating and/or preventing a uterine tumor described above in the manufacture of a medicament, health product, food, food additive.

A method of designing a substance for treating and/or preventing a prostate tumor, wherein, a stable structure corresponding to a prostate-tumor-related substance is used as the substance for treating and/or preventing the prostate tumor.

Preferably, the stable structure corresponding to the substance is a structure of the substance in solid form under high temperature over 500°C.

Preferably, the stable structure corresponding to the prostate-tumor-related substance is selected from one or more stable structure of the group consisting of:
(1) a stable structure corresponding to a substance that causes a prostate tumor lesion in an organism or the corresponding substance thereof that does not cause a prostate tumor lesion;
(2) a stable structure corresponding to the cells, tissues or organs where the prostate tumor occurs;
(3) a stable structure corresponding to the healthy cell, tissue or organ corresponding to the primary site of the prostate tumor;
(4) a stable structure corresponding to a substance containing the substance in any of the above (1) to (3).

A substance for treating and/or preventing the prostate tumor designed according to the method described above.

A method of preparing the substance for treating and/or preventing a prostate tumor described above:
the stable structure corresponding to the prostate-tumor-related substance is prepared by high-temperature carbonization.

Use of the substance for treating and/or preventing a prostate tumor described above in the manufacture of a medicament, health product, food, food additive.

A method of designing a substance for treating and/or preventing an ovarian tumor, wherein, a stable structure corresponding to an ovarian-tumor-related substance is used as the substance for treating and/or preventing the ovarian tumor.

Preferably, the stable structure corresponding to the substance is a structure of the substance in solid form under high temperature over 500°C.

Preferably, the stable structure corresponding to the ovarian-tumor-related substance is selected from one or more stable structure of the group consisting of:
(1) a stable structure corresponding to a substance that causes a ovarian tumor lesion in an organism or the corresponding substance thereof that does not cause a ovarian tumor lesion;
(2) a stable structure corresponding to the cells, tissues or organs where the ovarian tumor occurs;
(3) a stable structure corresponding to the healthy cell, tissue or organ corresponding to the primary site of the ovarian tumor;
(4) a stable structure corresponding to a substance containing the substance in any of the above (1) to (3).

A substance for treating and/or preventing the ovarian tumor designed according to the method described above.

A method of preparing the substance for treating and/or preventing an ovarian tumor described above:
the stable structure corresponding to the ovarian-tumor-related substance is prepared by high-temperature carbonization.

Use of the substance for treating and/or preventing an ovarian tumor described above in the manufacture of a medicament, health product, food, food additive.

A method of designing a substance for treating and/or preventing a lymphoma, wherein, a stable structure corresponding to a lymphoma-related substance is used as the substance for treating and/or preventing the lymphoma.

Preferably, the stable structure corresponding to the substance is a structure of the substance in solid form under high temperature over 500°C.

Preferably, the stable structure corresponding to the lymphoma-related substance is selected from one or more stable structure of the group consisting of:
(1) a stable structure corresponding to a substance that causes a lymphoma lesion in an organism or the corresponding substance thereof that does not cause a lymphoma lesion;
(2) a stable structure corresponding to the cells, tissues or organs where the lymphoma occurs;
(3) a stable structure corresponding to the healthy cell, tissue or organ corresponding to the primary site of the lymphoma;
(4) a stable structure corresponding to a substance containing the substance in any of the above (1) to (3).

A substance for treating and/or preventing the lymphoma designed according to the method described above.

A method of preparing the substance for treating and/or preventing a lymphoma described above:
the stable structure corresponding to the lymphoma-related substance is prepared by high-temperature carbonization.

Use of the substance for treating and/or preventing a lymphoma described above in the manufacture of a medicament, health product, food, food additive.

A method of designing a substance for treating and/or preventing a skin tumor, wherein, a stable structure corresponding to a skin-tumor-related substance is used as the substance for treating and/or preventing the skin tumor.

Preferably, the stable structure corresponding to the substance is a structure of the substance in solid form under high temperature over 500°C.

Preferably, the stable structure corresponding to the skin tumor-related substance is selected from one or more stable structure of the group consisting of:
(1) a stable structure corresponding to a substance that causes a skin tumor lesion in an organism or the corresponding substance thereof that does not cause a skin tumor lesion;
(2) a stable structure corresponding to the cells, tissues or organs where the skin tumor occurs;
(3) a stable structure corresponding to the healthy cell, tissue or organ corresponding to the primary site of the skin tumor;
(4) a stable structure corresponding to a substance containing the substance in any of the above (1) to (3).

A substance for treating and/or preventing the skin tumor designed according to the method described above.

A method of preparing the substance for treating and/or preventing a skin tumor described above:
the stable structure corresponding to the skin-tumor-related substance is prepared by high-temperature carbonization.

Use of the substance for treating and/or preventing a skin tumor described above in the manufacture of a medicament, health product, food, food additive.

A method of designing a substance for treating and/or preventing a thyroid tumor, wherein, a stable structure corresponding to a thyroid-tumor-related substance is used as the substance for treating and/or preventing the thyroid tumor.

Preferably, the stable structure corresponding to the substance is a structure of the substance in solid form under high temperature over 500°C.

Preferably, the stable structure corresponding to the thyroid-tumor-related substance is selected from one or more stable structure of the group consisting of:
(1) a stable structure corresponding to a substance that causes a thyroid tumor lesion in an organism or the corresponding substance thereof that does not cause a thyroid tumor lesion;
(2) a stable structure corresponding to the cells, tissues or organs where the thyroid tumor occurs;
(3) a stable structure corresponding to the healthy cell, tissue or organ corresponding to the primary site of the thyroid tumor;
(4) a stable structure corresponding to a substance containing the substance in any of the above (1) to (3).

A substance for treating and/or preventing the thyroid tumor designed according to the method described above.

A method of preparing the substance for treating and/or preventing a thyroid tumor described above:
the stable structure corresponding to the thyroid-tumor-related substance is prepared by high-temperature carbonization.

Use of the substance for treating and/or preventing a thyroid tumor described above in the manufacture of a medicament, health product, food, food additive.

A method of designing a substance for treating and/or preventing a mesenchymal tissue tumor, wherein, a stable structure corresponding to a mesenchymal-tissue-tumor-related substance is used as the substance for treating and/or preventing the mesenchymal tissue tumor.

Preferably, the stable structure corresponding to the substance is a structure of the substance in solid form under high temperature over 500°C.

Preferably, the stable structure corresponding to the mesenchymal-tissue-tumor-related substance is selected from one or more stable structure of the group consisting of:
a stable structure corresponding to a substance that causes a mesenchymal tissue tumor lesion in an organism or the corresponding substance thereof that does not cause a mesenchymal tissue tumor lesion;
(2) a stable structure corresponding to the cells, tissues or organs where the mesenchymal tissue tumor occurs;
(3) a stable structure corresponding to the healthy cell, tissue or organ corresponding to the primary site of the mesenchymal tissue tumor;
(4) a stable structure corresponding to a substance containing the substance in any of the above (1) to (3).

A substance for treating and/or preventing the mesenchymal tissue tumor designed according to the method described above.

A method of preparing the substance for treating and/or preventing a mesenchymal tissue tumor described above:
the stable structure corresponding to the mesenchymal-tissue-tumor-related substance is prepared by high-temperature carbonization.

Use of the substance for treating and/or preventing a mesenchymal tissue tumor described above in the manufacture of a medicament, health product, food, food additive.

A method of designing a substance for treating and/or preventing rhabdomyosarcoma, wherein, a stable structure corresponding to a rhabdomyosarcoma-related substance is used as the substance for treating and/or preventing the rhabdomyosarcoma.

Preferably, the stable structure corresponding to the substance is a structure of the substance in solid form under high temperature over 500°C.

Preferably, the stable structure corresponding to the rhabdomyosarcoma-related substance is selected from one or more stable structure of the group consisting of:
(1) a stable structure corresponding to a substance that causes a rhabdomyosarcoma lesion in an organism or the corresponding substance thereof that does not cause a rhabdomyosarcoma lesion;
(2) a stable structure corresponding to the cells, tissues or organs where the rhabdomyosarcoma occurs;
(3) a stable structure corresponding to the healthy cell, tissue or organ corresponding to the primary site of the rhabdomyosarcoma;
(4) a stable structure corresponding to a substance containing the substance in any of the above (1) to (3).

A substance for treating and/or preventing the rhabdomyosarcoma designed according to the method described above.

A method of preparing the substance for treating and/or preventing rhabdomyosarcoma described above:
the stable structure corresponding to the rhabdomyosarcoma-related substance is prepared by high-temperature carbonization.

Use of the substance for treating and/or preventing rhabdomyosarcoma described above in the manufacture of a medicament, health product, food, food additive.

A method of designing a substance for treating and/or preventing lipoma, wherein, a stable structure corresponding to a lipoma-related substance is used as the substance for treating and/or preventing the lipoma.

Preferably, the stable structure corresponding to the substance is a structure of the substance in solid form under high temperature over 500°C.

Preferably, the stable structure corresponding to the lipoma-related substance is selected from one or more stable structure of the group consisting of:
(1) a stable structure corresponding to a substance that causes a lipoma lesion in an organism or the corresponding substance thereof that does not cause a lipoma lesion;
(2) a stable structure corresponding to the cells, tissues or organs where the lipoma occurs;
(3) a stable structure corresponding to the healthy cell, tissue or organ corresponding to the primary site of the lipoma;
(4) a stable structure corresponding to a substance containing the substance in any of the above (1) to (3).

A substance for treating and/or preventing the lipoma designed according to the method described above.

A method of preparing the substance for treating and/or preventing lipoma described above:
the stable structure corresponding to the lipoma-related substance is prepared by high-temperature carbonization.

Use of the substance for treating and/or preventing lipoma described above in the manufacture of a medicament, health product, food, food additive.

A method of designing a substance for treating and/or preventing osteosarcoma, wherein, a stable structure corresponding to an osteosarcoma-related substance is used as the substance for treating and/or preventing the osteosarcoma.

Preferably, the stable structure corresponding to the substance is a structure of the substance in solid form under high temperature over 500°C.

Preferably, the stable structure corresponding to the osteosarcoma-related substance is selected from one or more stable structure of the group consisting of:
(1) a stable structure corresponding to a substance that causes an osteosarcoma lesion in an organism or the corresponding substance thereof that does not cause an osteosarcoma lesion;
(2) a stable structure corresponding to the cells, tissues or organs where the osteosarcoma occurs;
(3) a stable structure corresponding to the healthy cell, tissue or organ corresponding to the primary site of the osteosarcoma;
(4) a stable structure corresponding to a substance containing the substance in any of the above (1) to (3).

A substance for treating and/or preventing the osteosarcoma designed according to the method described above.

A method of preparing the substance for treating and/or preventing osteosarcoma described above:
the stable structure corresponding to the osteosarcoma-related substance is prepared by high-temperature carbonization.

Use of the substance for treating and/or preventing osteosarcoma described above in the manufacture of a medicament, health product, food, food additive.

A method of designing a substance for treating and/or preventing hemangioma, wherein, a stable structure corresponding to a hemangioma-related substance is used as the substance for treating and/or preventing the hemangioma.

Preferably, the stable structure corresponding to the substance is a structure of the substance in solid form under high temperature over 500°C.

Preferably, the stable structure corresponding to the hemangioma-related substance is selected from one or more stable structure of the group consisting of:
(1) a stable structure corresponding to a substance that causes a hemangioma lesion in an organism or the corresponding substance thereof that does not cause a hemangioma lesion;
(2) a stable structure corresponding to the cells, tissues or organs where the hemangioma occurs;
(3) a stable structure corresponding to the healthy cell, tissue or organ corresponding to the primary site of the hemangioma;
(4) a stable structure corresponding to a substance containing the substance in any of the above (1) to (3).

A substance for treating and/or preventing the hemangioma designed according to the method described above.

A method of preparing the substance for treating and/or preventing hemangioma described above:
the stable structure corresponding to the hemangioma-related substance is prepared by high-temperature carbonization.

Use of the substance for treating and/or preventing hemangioma described above in the manufacture of a medicament, health product, food, food additive.

A method of designing a substance for treating and/or preventing lymphangioma, wherein, a stable structure corresponding to a lymphangioma-related substance is used as the substance for treating and/or preventing the lymphangioma.

Preferably, the stable structure corresponding to the substance is a structure of the substance in solid form under high temperature over 500°C.

Preferably, the stable structure corresponding to the lymphangioma-related substance is selected from one or more stable structure of the group consisting of:
(1) a stable structure corresponding to a substance that causes a lymphangioma lesion in an organism or the corresponding substance thereof that does not cause a lymphangioma lesion;
(2) a stable structure corresponding to the cells, tissues or organs where the lymphangioma occurs;
(3) a stable structure corresponding to the healthy cell, tissue or organ corresponding to the primary site of the lymphangioma;
(4) a stable structure corresponding to a substance containing the substance in any of the above (1) to (3).

A substance for treating and/or preventing the lymphangioma designed according to the method described above.

A method of preparing the substance for treating and/or preventing lymphangioma described above:
the stable structure corresponding to the lymphangioma-related substance is prepared by high-temperature carbonization.

Use of the substance for treating and/or preventing lymphangioma described above in the manufacture of a medicament, health product, food, food additive.

A method of designing a substance for treating and/or preventing leiomyoma of intestinal wall, wherein, a stable structure corresponding to a substance related to leiomyoma of intestinal wall is used as the substance for treating and/or preventing the rhabdomyosarcoma.

Preferably, the stable structure corresponding to the substance is a structure of the substance in solid form under high temperature over 500°C .

Preferably, the stable structure corresponding to the substance related to leiomyoma of intestinal wall is selected from one or more stable structure of the group consisting of:
(1) a stable structure corresponding to a substance related to leiomyoma lesion of intestinal wall in an organism or the corresponding substance thereof that does not cause a leiomyoma lesion of intestinal wall;
(2) a stable structure corresponding to the cells, tissues or organs where the leiomyoma of intestinal wall occurs;
(3) a stable structure corresponding to the healthy cell, tissue or organ corresponding to the primary site of the leiomyoma of intestinal wall;
(4) a stable structure corresponding to a substance containing the substance in any of the above (1) to (3).

A substance for treating and/or preventing the leiomyoma of intestinal wall designed according to the method described above.

A method of preparing the substance for treating and/or preventing leiomyoma of intestinal wall described above:
the stable structure corresponding to the substance related to leiomyoma of intestinal wall is prepared by high-temperature carbonization.

Use of the substance for treating and/or preventing leiomyoma of intestinal wall described above in the manufacture of a medicament, health product, food, food additive.

A method of designing a substance for treating and/or preventing periosteal chondroma, wherein, a stable structure corresponding to a periosteal-chondroma-related substance is used as the substance for treating and/or preventing the periosteal chondroma.

Preferably, the stable structure corresponding to the substance is a structure of the substance in solid form under high temperature over 500°C.

Preferably, the stable structure corresponding to the periosteal-chondroma-related substance is selected from one or more stable structure of the group consisting of:
(1) a stable structure corresponding to a substance that causes an periosteal chondroma lesion in an organism or the corresponding substance thereof that does not cause an periosteal chondroma lesion;
(2) a stable structure corresponding to the cells, tissues or organs where the periosteal chondroma occurs;
(3) a stable structure corresponding to the healthy cell, tissue or organ corresponding to the primary site of the periosteal chondroma;
(4) a stable structure corresponding to a substance containing the substance in any of the above (1) to (3).

A substance for treating and/or preventing the periosteal chondroma designed according to the method described above.

A method of preparing the substance for treating and/or preventing periosteal chondroma described above:
the stable structure corresponding to the periosteal-chondroma-related substance is prepared by high-temperature carbonization.

Use of the substance for treating and/or preventing periosteal chondroma described above in the manufacture of a medicament, health product, food, food additive.

A method of designing a substance for treating and/or preventing a neuroectodermal tumor, wherein, a stable structure corresponding to a neuroectodermal-tumor-related substance is used as the substance for treating and/or preventing the neuroectodermal tumor.

Preferably, the stable structure corresponding to the substance is a structure of the substance in solid form under high temperature over 500°C.

Preferably, the stable structure corresponding to the neuroectodermal-tumor-related substance is selected from one or more stable structure of the group consisting of:
(1) a stable structure corresponding to a substance that causes a neuroectodermal tumor lesion in an organism or the corresponding substance thereof that does not cause a neuroectodermal tumor lesion;
(2) a stable structure corresponding to the cells, tissues or organs where the neuroectodermal tumor occurs;
(3) a stable structure corresponding to the healthy cell, tissue or organ corresponding to the primary site of the neuroectodermal tumor;
(4) a stable structure corresponding to a substance containing the substance in any of the above (1) to (3).

A substance for treating and/or preventing the neuroectodermal tissue tumor designed according to the method described above.

A method of preparing the substance for treating and/or preventing a neuroectodermal tissue tumor described above:
the stable structure corresponding to the neuroectodermal-tumor-related substance is prepared by high-temperature carbonization.

Use of the substance for treating and/or preventing a neuroectodermal tumor described above in the manufacture of a medicament, health product, food, food additive.

A method of designing a substance for treating and/or preventing an intracranial tumor, wherein, a stable structure corresponding to an intracranial-tumor-related substance is used as the substance for treating and/or preventing the intracranial tumor.

Preferably, the stable structure corresponding to the substance is a structure of the substance in solid form under high temperature over 500°C.

Preferably, the stable structure corresponding to the intracranial-tumor-related substance is selected from one or more stable structure of the group consisting of:
(1) a stable structure corresponding to a substance that causes an intracranial tumor lesion in an organism or the corresponding substance thereof that does not cause an intracranial tumor lesion;
(2) a stable structure corresponding to the cells, tissues or organs where the intracranial tumor occurs;
(3) a stable structure corresponding to the healthy cell, tissue or organ corresponding to the primary site of the intracranial tumor;
(4) a stable structure corresponding to a substance containing the substance in any of the above (1) to (3).

A substance for treating and/or preventing the intracranial tumor designed according to the method described above.

A method of preparing the substance for treating and/or preventing an intracranial tumor described above:
the stable structure corresponding to the intracranial-tumor-related substance is prepared by high-temperature carbonization.

Use of the substance for treating and/or preventing an intracranial tumor described above in the manufacture of a medicament, health product, food, food additive.

A method of designing a substance for treating and/or preventing schwannoma, wherein, a stable structure corresponding to a schwannoma-related substance is used as the substance for treating and/or preventing the schwannoma.

Preferably, the stable structure corresponding to the substance is a structure of the substance in solid form under high temperature over 500°C.

Preferably, the stable structure corresponding to the schwannoma-related substance is selected from one or more stable structure of the group consisting of:
(1) a stable structure corresponding to a substance that causes a schwannoma lesion in an organism or the corresponding substance thereof that does not cause a schwannoma lesion;
(2) a stable structure corresponding to the cells, tissues or organs where the schwannoma occurs;
(3) a stable structure corresponding to the healthy cell, tissue or organ corresponding to the primary site of the schwannoma;
(4) a stable structure corresponding to a substance containing the substance in any of the above (1) to (3).

A substance for treating and/or preventing the schwannoma designed according to the method described above.

A method of preparing the substance for treating and/or preventing schwannoma described above:
the stable structure corresponding to the schwannoma-related substance is prepared by high-temperature carbonization.

Use of the substance for treating and/or preventing schwannoma described above in the manufacture of a medicament, health product, food, food additive.

A method of designing a substance for treating and/or preventing neurofibroma, wherein, a stable structure corresponding to a neurofibroma-related substance is used as the substance for treating and/or preventing the neurofibroma.

Preferably, the stable structure corresponding to the substance is a structure of the substance in solid form under high temperature over 500°C.

Preferably, the stable structure corresponding to the neurofibroma-related substance is selected from one or more stable structure of the group consisting of:
(1) a stable structure corresponding to a substance that causes a neurofibroma lesion in an organism or the corresponding substance thereof that does not cause a neurofibroma lesion;
(2) a stable structure corresponding to the cells, tissues or organs where the neurofibroma occurs;
(3) a stable structure corresponding to the healthy cell, tissue or organ corresponding to the primary site of the neurofibroma;
(4) a stable structure corresponding to a substance containing the substance in any of the above (1) to (3).

A substance for treating and/or preventing the neurofibroma designed according to the method described above.

A method of preparing the substance for treating and/or preventing neurofibroma described above:
the stable structure corresponding to the neurofibroma-related substance is prepared by high-temperature carbonization.

Use of the substance for treating and/or preventing neurofibroma described above in the manufacture of a medicament, health product, food, food additive.

A method of designing a substance for treating and/or preventing retinoblastoma, wherein, a s stable structure corresponding to a retinoblastoma-related substance is used as the substance for treating and/or preventing the retinoblastoma.

Preferably, the stable structure corresponding to the substance is a structure of the substance in solid form under high temperature over 500°C.

Preferably, the stable structure corresponding to the retinoblastoma-related substance is selected from one or more stable structure of the group consisting of:
(1) a stable structure corresponding to a substance that causes a retinoblastoma lesion in an organism or the corresponding substance thereof that does not cause a retinoblastoma lesion;
(2) a stable structure corresponding to the cells, tissues or organs where the retinoblastoma occurs;
(3) a stable structure corresponding to the healthy cell, tissue or organ corresponding to the primary site of the retinoblastoma;
(4) a stable structure corresponding to a substance containing the substance in any of the above (1) to (3).

A substance for treating and/or preventing the retinoblastoma designed according to the method described above.

A method of preparing the substance for treating and/or preventing retinoblastoma described above:
the stable structure corresponding to the retinoblastoma-related substance is prepared by high-temperature carbonization.

Use of the substance for treating and/or preventing retinoblastoma described above in the manufacture of a medicament, health product, food, food additive.

A method of designing a substance for treating and/or preventing malignant melanoma, wherein, a stable structure corresponding to a malignant-melanoma-related substance is used as the substance for treating and/or preventing the malignant melanoma.

Preferably, the stable structure corresponding to the substance is a structure of the substance in solid form under high temperature over 500°C.

Preferably, the stable structure corresponding to the malignant-melanoma-related substance is selected from one or more stable structure of the group consisting of:
(1) a stable structure corresponding to a substance that causes a malignant melanoma lesion in an organism or the corresponding substance thereof that does not cause a malignant melanoma lesion;
(2) a stable structure corresponding to the cells, tissues or organs where the malignant melanoma occurs;
(3) a stable structure corresponding to the healthy cell, tissue or organ corresponding to the primary site of the malignant melanoma;
(4) a stable structure corresponding to a substance containing the substance in any of the above (1) to (3).

A substance for treating and/or preventing the malignant melanoma designed according to the method described above.

A method of preparing the substance for treating and/or preventing malignant melanoma described above:
the stable structure corresponding to the malignant-melanoma-related substance is prepared by high-temperature carbonization.

Use of the substance for treating and/or preventing malignant melanoma described above in the manufacture of a medicament, health product, food, food additive.

A method of designing a substance for treating and/or preventing leukemia, wherein, a stable structure corresponding to a leukemia-related substance is used as the substance for treating and/or preventing the leukemia.

Preferably, the stable structure corresponding to the substance is a structure of the substance in solid form under high temperature over 500°C.

Preferably, the stable structure corresponding to the leukemia-related substance is selected from one or more stable structure of the group consisting of:
(1) a stable structure corresponding to a substance that causes leukemia lesion in an organism or the corresponding substance thereof that does not cause leukemia lesion;
(2) a stable structure corresponding to the cells, tissues or organs where leukemia occurs;
(3) a stable structure corresponding to the healthy cell, tissue or organ corresponding to the primary site of the leukemia;
(4) a stable structure corresponding to a substance containing the substance in any of the above (1) to (3).

A substance for treating and/or preventing the leukemia designed according to the method described above.

A method of preparing the substance for treating and/or preventing leukemia described above:
the stable structure corresponding to the leukemia-related substance is prepared by high-temperature carbonization.

Use of the substance for treating and/or preventing leukemia described above in the manufacture of a medicament, health product, food, food additive.

A method of designing a substance for treating and/or preventing spermatocytoma, wherein, a stable structure corresponding to a spermatocytoma-related substance is used as the substance for treating and/or preventing the spermatocytoma.

Preferably, the stable structure corresponding to the substance is a structure of the substance in solid form under high temperature over 500°C.

Preferably, the stable structure corresponding to the spermatocytoma-related substance is selected from one or more stable structure of the group consisting of:
(1) a stable structure corresponding to a substance that causes a spermatocytoma lesion in an organism or the corresponding substance thereof that does not cause a spermatocytoma lesion;
(2) a stable structure corresponding to the cells, tissues or organs where the spermatocytoma occurs;
(3) a stable structure corresponding to the healthy cell, tissue or organ corresponding to the primary site of the spermatocytoma;
(4) a stable structure corresponding to a substance containing the substance in any of the above (1) to (3).

A substance for treating and/or preventing the spermatocytoma designed according to the method described above.

A method of preparing the substance for treating and/or preventing spermatocytoma described above:
the stable structure corresponding to the spermatocytoma-related substance is prepared by high-temperature carbonization.

Use of the substance for treating and/or preventing spermatocytoma described above in the manufacture of a medicament, health product, food, food additive.

A method of designing a substance for treating and/or preventing a granulosa cell tumor, wherein, a stable structure corresponding to a substance related to the granulosa cell tumor is used as the substance for treating and/or preventing the granulosa cell tumor.

Preferably, the stable structure corresponding to the substance is a structure of the substance in solid form under high temperature over 500°C.

Preferably, the stable structure corresponding to the substance related to the granulosa cell tumor is selected from one or more stable structure of the group consisting of:
(1) a stable structure corresponding to a substance that causes a granulosa cell tumor lesion in an organism or the corresponding substance thereof that does not cause a granulosa cell tumor lesion;
(2) a stable structure corresponding to the cells, tissues or organs where the granulosa cell tumor occurs;
(3) a stable structure corresponding to the healthy cell, tissue or organ corresponding to the primary site of the granulosa cell tumor;
(4) a stable structure corresponding to a substance containing the substance in any of the above (1) to (3).

A substance for treating and/or preventing the granulosa cell tumor designed according to the method described above.

A method of preparing the substance for treating and/or preventing a granulosa cell tumor described above:
the stable structure corresponding to the substance related to the granulosa cell tumor is prepared by high-temperature carbonization.

Use of the substance for treating and/or preventing a granulosa cell tumor described above in the manufacture of a medicament, health product, food, food additive.

A method of designing a substance for treating and/or preventing Ewing's sarcoma, wherein, a stable structure corresponding to Ewing's-sarcoma-related substance is used as the substance for treating and/or preventing the Ewing's sarcoma.

Preferably, the stable structure corresponding to the substance is a structure of the substance in solid form under high temperature over 500°C .

Preferably, the stable structure corresponding to the Ewing's-sarcoma-related substance is selected from one or more stable structure of the group consisting of:
(1) a stable structure corresponding to a substance that causes an Ewing's sarcoma lesion in an organism or the corresponding substance thereof that does not cause an Ewing's sarcoma lesion;
(2) a stable structure corresponding to the cells, tissues or organs where the Ewing's sarcoma occurs;
(3) a stable structure corresponding to the healthy cell, tissue or organ corresponding to the primary site of the Ewing's sarcoma;
(4) a stable structure corresponding to a substance containing the substance in any of the above (1) to (3).

A substance for treating and/or preventing the Ewing's sarcoma designed according to the method described above.

A method of preparing the substance for treating and/or preventing Ewing's sarcoma described above:
the stable structure corresponding to the Ewing's-sarcoma-related substance is prepared by high-temperature carbonization.

Use of the substance for treating and/or preventing Ewing's sarcoma described above in the manufacture of a medicament, health product, food, food additive.

The essence of health is the dynamic balance of the structural system. This dynamic balance is the result of the interaction among the structures in the structural system. The most basic interaction is promotion and restriction. In an organism structural system, if a structure lacks a structure that promotes it, this structure will be insufficient; and if a structure lacks a structure that restricts it, this structure will be too much. These will cause an imbalance in the structural system. The essence of disease is the imbalance of organism structure system. It can be seen from the above technical solutions that, in the method for treating or preventing diseases disclosed in the present disclosure, a stable structure of disease-related substances is used as the external structure which applies on the organism structural system (such as oral administration), and the organism structural system regulates genes or the expression of genes, and treats or prevents diseases related to the substance through processes such as the self-adaptation and self-organization after the recognition and transmission of the structural information of the external structure.

It can be seen from the above technical solutions that the substance for treating and/or preventing a disease disclosed in the present disclosure and the design method and preparation method thereof have beneficial effects including:
1. The design method and preparation method of the substance for treating and/or preventing diseases of the present disclosure are not based on the specific structure of the disease-causing substance, not the specific mechanism of the disease, but on the stable structure corresponding to the same or similar substance as the external substance or internal substance that causes diseases. Such stable structure is used as the external structure to apply on the organism structural system. The external structure can enable the organism to eliminate, alleviate, or prevent diseases or the occurrence of diseases according to self-adaptation and self-organization of structures. Therefore, the method is versatile.
2. The present disclosure treat and/or prevent diseases from the perspective of biological systems by combining the specific structure of Western medicine research and the macro theory established by TCM. Relatively speaking, the system is limited, while the disease is infinite. Different lesions of the same organ can lead to different diseases, and the symptoms of the same disease in different individuals are not exactly the same. Systemically, humans are the same according to either Western medicine or TCM, even humans and swine, cattle, sheep, primates are basically the same. Therefore, for diseases caused by internal lesions of organisms, designing substances for the treatment and/or prevention of diseases according to the system only need the specific diseased cells, tissues or organs. There is no need to explore the mechanism through which the disease is caused, or the specific symptoms of the disease. Only the system where the disease-related substances are located, or the cells, tissues or organs in the system are needed. Upon applying its corresponding stable structure as an external structure on the organism structural system, the organism structural system can make the organism establish a balance with the structure of the external substance or restore the inherent structural imbalance of the organism structural system according to the self-adaptation and self-organization of structures, thereby eliminating, alleviating, or preventing the occurrence of diseases.
3. The substance for treating and/or preventing diseases and its design method and preparation method of the present disclosure avoid the research on the disease mechanism and greatly save time, labor, material resources, and financial resources. It greatly shortens the time of drug research and development and reduces the cost of disease treatment, which is the ultimate simplification of drug research and development, disease diagnosis and treatment.

### DETAILED DESCRIPTION

The present disclosure relates to a substance for treating and/or preventing tumors, and design method and preparation method thereof. Those skilled in the art can learn from the content of this article, can use different materials, can improve the process or use other similar process, which are all considered to be included in the present disclosure. In particular, it should be noted that all similar substitutions and modifications are obvious to those skilled in the art, which are all deemed to be included in the present disclosure. The method and product of the present disclosure have been described in the preferred embodiments. It is obvious that the skilled in the art can make appropriate changes and combinations to the methods described herein without departing from the spirit and scope of the present disclosure to implement the present disclosure.

In order to further understand the present disclosure, the technical solutions in the embodiments of the present disclosure will be clearly and completely described below in conjunction with the Examples. It is understand that the described Examples are only a part of the embodiments of the present disclosure, rather than all the embodiments. Based on the Examples of the present disclosure, all other embodiments obtained by those of ordinary skill in the art without creative work shall fall within the scope of the present disclosure.

The specific Examples are as follows:

### Example 1: substance for treating and/or preventing lung tumors and method of preparing the substance

Tumors are gene mutations or abnormal gene expression, which are essentially imbalances of the structural system established by abstract and concrete structures. For example, when the nucleus of a cancer cell is transplanted into a normal fertilized egg, because the genes are in a normal and balanced structural environment, the expression of the genes can return to normal, and the egg can develop into a healthy organism. Therefore, by treating with a normal external structure, the expression of cancer genes can be restored to normal, so that tumor cells can develop into normal cells. Every structure in normal lung cells has a structure that interacts with it, and this interaction achieves a dynamic balance. Such dynamic balance can be disrupted by lung cancer cells. A stable structure corresponding to normal lung cells can be used as the external structure to apply on the organism structural system. Due to the self-adaptive, self-organizing, and self-stabilizing functions of the organism structural system, the organism structural system regulates the expression of genes in structures that interacts with the external structure to standard levels according to the structural information of each structure in the external structure. That is, the structural environment of normal lung cells is used to affect the gene expression of lung cancer cells to restore the dynamic balance of the structures. In this Example, healthy swine lungs are carbonized at high temperature to obtain substances for treating and preventing lung tumors.

### Example 2: Clinical application of the substance prepared in Example 1 on the treatment of lung cancer

This Example uses the substance prepared in Example 1 above for efficacy verification. Details are as follows:
1. Clinical data: A total of 24 patients with lung cancer were recruited, including 12 males and 12 females, aged 45-79 years old. The patients themselves before the treatment were used as controls.
2. Diagnosis criteria: Referring to the diagnosis and staging criteria of lung cancer in "Modern Oncology".
3. Treatment: Orally administering to the patient the substance prepared in Example 1 once per day and 3 g each time for continuous 10 days.
4. Efficacy evaluation criteria: Complete remission, all the lesions in the body disappear completely for >4 weeks; partial remission, the sum of the longest diameters of the lesions reduces by >30% for ≥4 weeks; stable disease, the original lesions do not changed significantly and no new lesion appears; progressive disease, the sum of the longest diameters of the lesions increases by ≥20%, and the absolute value increases by >5mm, or new lesions appear. Total effective rate = (complete remission + partial remission)/total case number x 100%.
5. Result: After treatment, 18 patients showed complete remission, 2 patients showed partial remission, and 4 patients showed stable disease. The total effective rate was 83.33%.

### Example 3: substance for treating and/or preventing liver tumors and method of preparing the substance

In this Example, healthy swine livers are carbonized at high temperature to obtain substances for treating and preventing liver tumors.

### Example 4: Clinical application of the substance prepared in Example 3 on the treatment of liver cancer

This Example uses the substance prepared in Example 3 above for efficacy verification. Details are as follows:
1. Clinical data: A total of 10 patients aged 31-80 years old with liver cancer were recruited. The patients themselves before the treatment were used as controls.
2. Diagnosis criteria: (1) Excluding patients who have active liver disease, pregnancy, germline embryogenic tumors, metastatic liver cancer, hard liver, liver with a mass, or patients who have characteristic space-occupying lesions in liver as shown by imaging examination; (2) patients who have characteristic space-occupying lesions in liver as shown by two imaging examinations, or patients who have two positive liver cancer markers and characteristic space-occupying lesions in liver as shown by one imaging examination.
3. Treatment: Orally administering to the patient the substance prepared in Example 3 once per day and 3 g each time for continuous 10 days.
4. Efficacy evaluation criteria: complete remission, baseline lesions disappear for 4 weeks; partial remission, the sum of the long diameters of baseline lesions reduces by ≥30% for 4 weeks; stable disease, the sum of the long diameters of baseline lesions reduces but does not reach the level of partial remission, or increases but does not reach the level of progressive disease; progressive disease, the sum of the long diameters of baseline lesions increases by ≥20%, or new lesions appear. Total effective rate = (complete remission + partial remission)/total case number x 100%.
5. Result: 6 patients showed complete remission, 3 patients showed partial remission, and 1 patients showed stable disease. The total effective rate was 90.00%.

### Example 5: substance for treating and/or preventing gastric tumors and method of preparing the substance

In this Example, healthy swine stomachs are carbonized at high temperature to obtain substances for treating and preventing gastric tumors.

### Example 6: Clinical application of the substance prepared in Example 5 on the treatment of lung cancer

This Example uses the substance prepared in Example 5 above for efficacy verification. Details are as follows:
1. Clinical data: A total of 11 patients aged 28-70 years old with gastric cancer were recruited. The patients themselves before the treatment were used as controls.
2. Diagnostic criteria: Referring to the diagnostic criteria for gastric cancer in the "Health Industry Standards of the People's Republic of China: Diagnostic Criteria for Gastric Cancer (WS 316-2010)" .
3. Treatment: Orally administering to the patient the substance prepared in Example 5 once per day and 3 g each time for continuous 10 days, for one week.
4. Efficacy evaluation criteria: complete remission, lesions disappear for at least 4 weeks; partial remission, the sum of the longest diameters of target lesions reduces by ≥50% for 4 weeks, no new lesion appears; stable disease, the lesions do not significantly change compared with those before the treatment, and no new lesion appears; progressive disease, the sum of the longest diameters of target lesions increases by ≥25%, or the new lesions are malignant. Total effective rate = (complete remission + partial remission)/total case number x 100%.
5. Result: after the treatment, 6 patients showed complete remission, 3 patients showed partial remission, and 2 patients showed stable disease. The total effective rate was 81.82%.

## Claims

1. A method of designing a substance for treating and/or preventing a tumor, wherein,
a stable structure corresponding to a tumor-related substance is used as the substance for treating and/or preventing the tumor;
the stable structure corresponding to the substance is a structure of the substance in solid form under high temperature over 500°C.

2. The method according to claim 1, wherein, the stable structure corresponding to the tumor-related substance is selected from one or more stable structure of the group consisting of:
(1)a stable structure corresponding to a substance that causes a tumor lesion in an organism or the corresponding substance thereof that does not cause a tumor lesion;
(2)a stable structure corresponding to the cells, tissues or organs where the tumor occurs;
(3)a stable structure corresponding to the healthy cell, tissue or organ corresponding to the primary site of the tumor;
(4)a stable structure corresponding to a substance containing the substance in any of the above (1) to (3).

3. A substance for treating and/or preventing the tumor designed according to the method of claims 1 or 2.

4. A method of preparing the substance for treating and/or preventing a tumor according to claim 3, wherein,
the stable structure corresponding to the substance is prepared by high-temperature carbonization.

5. A method of designing a substance for treating and/or preventing an epithelial tissue tumor, wherein,
a stable structure corresponding to an epithelial-tissue-tumor-related substance is used as the substance for treating and/or preventing the epithelial tissue tumor;
the stable structure corresponding to the substance is a structure of the substance in solid form under high temperature over 500°C.

6. The method according to claim 5, wherein, the stable structure corresponding to the epithelial-tissue-tumor-related substance is selected from one or more stable structure of the group consisting of:
(1) a stable structure corresponding to a substance that causes an epithelial tissue tumor lesion in an organism or the corresponding substance thereof that does not cause an epithelial tissue tumor lesion;
(2) a stable structure corresponding to the cells, tissues or organs where the epithelial tissue tumor occurs;
(3) a stable structure corresponding to the healthy cell, tissue or organ corresponding to the primary site of the epithelial tissue tumor;
(4) a stable structure corresponding to a substance containing the substance in any of the above (1) to (3).

7. A substance for treating and/or preventing the epithelial tissue tumor designed according to the method of claims 5 or 6.

8. A method of preparing the substance for treating and/or preventing an epithelial tissue tumor according to claim 7, wherein,
the stable structure corresponding to the substance is prepared by high-temperature carbonization.

9. A method of designing a substance for treating and/or preventing a gastric tumor, wherein,
a stable structure corresponding to a gastric-tumor-related substance is used as the substance for treating and/or preventing the gastric tumor;
the stable structure corresponding to the substance is a structure of the substance in solid form under high temperature over 500°C.

10. The method according to claim 9, wherein, the stable structure corresponding to the gastric-tumor-related substance is selected from one or more stable structure of the group consisting of:
(1) a stable structure corresponding to a substance that causes a gastric tumor lesion in an organism or the corresponding substance thereof that does not cause a gastric tumor lesion;
(2) a stable structure corresponding to the cells, tissues or organs where the gastric tumor occurs;
(3) a stable structure corresponding to the healthy cell, tissue or organ corresponding to the primary site of the gastric tumor;
(4) a stable structure corresponding to a substance containing the substance in any of the above (1) to (3).

11. A substance for treating and/or preventing the gastric tumor designed according to the method of claims 9 or 10.

12. A method of preparing the substance for treating and/or preventing a gastric tumor according to claim 11, wherein,
the stable structure corresponding to the substance is prepared by high-temperature carbonization.

13. A method of designing a substance for treating and/or preventing a liver tumor, wherein,
a stable structure corresponding to a liver-tumor-related substance is used as the substance for treating and/or preventing the liver tumor;
the stable structure corresponding to the substance is a structure of the substance in solid form under high temperature over 500°C.

14. The method according to claim 13, wherein, the stable structure corresponding to the liver-tumor-related substance is selected from one or more stable structure of the group consisting of:
(1) a stable structure corresponding to a substance that causes a liver tumor lesion in an organism or the corresponding substance thereof that does not cause a liver tumor lesion;
(2) a stable structure corresponding to the cells, tissues or organs where the liver tumor occurs;
(3) a stable structure corresponding to the healthy cell, tissue or organ corresponding to the primary site of the liver tumor;
(4) a stable structure corresponding to a substance containing the substance in any of the above (1) to (3).

15. A substance for treating and/or preventing the liver tumor designed according to the method of claims 13 or 14.

16. A method of preparing the substance for treating and/or preventing a liver tumor according to claim 15, wherein,
the stable structure corresponding to the substance is prepared by high-temperature carbonization.

17. A method of designing a substance for treating and/or preventing a lung tumor, wherein,
a stable structure corresponding to a lung-tumor-related substance is used as the substance for treating and/or preventing the lung tumor;
the stable structure corresponding to the substance is a structure of the substance in solid form under high temperature over 500°C.

18. The method according to claim 17, wherein, the stable structure corresponding to the lung-tumor-related substance is selected from one or more stable structure of the group consisting of:
(1) a stable structure corresponding to a substance that causes a lung tumor lesion in an organism or the corresponding substance thereof that does not cause a lung tumor lesion;
(2) a stable structure corresponding to the cells, tissues or organs where the lung tumor occurs;
(3) a stable structure corresponding to the healthy cell, tissue or organ corresponding to the primary site of the lung tumor;
(4) a stable structure corresponding to a substance containing the substance in any of the above (1) to (3).

19. A substance for treating and/or preventing the lung tumor designed according to the method of claims 17 or 18.

20. A method of preparing the substance for treating and/or preventing a lung tumor according to claim 19, wherein,
the stable structure corresponding to the substance is prepared by high-temperature carbonization.

21. A method of designing a substance for treating and/or preventing a bladder tumor, wherein,
a stable structure corresponding to a bladder-tumor-related substance is used as the substance for treating and/or preventing the bladder tumor;
the stable structure corresponding to the substance is a structure of the substance in solid form under high temperature over 500°C.

22. The method according to claim 21, wherein, the stable structure corresponding to the bladder-tumor-related substance is selected from one or more stable structure of the group consisting of:
(1) a stable structure corresponding to a substance that causes a bladder tumor lesion in an organism or the corresponding substance thereof that does not cause a bladder tumor lesion;
(2) a stable structure corresponding to the cells, tissues or organs where the bladder tumor occurs;
(3) a stable structure corresponding to the healthy cell, tissue or organ corresponding to the primary site of the bladder tumor;
(4) a stable structure corresponding to a substance containing the substance in any of the above (1) to (3).

23. A substance for treating and/or preventing the bladder tumor designed according to the method of claims 21 or 22.

24. A method of preparing the substance for treating and/or preventing a bladder tumor according to claim 23, wherein,
the stable structure corresponding to the substance is prepared by high-temperature carbonization.

25. A method of designing a substance for treating and/or preventing a prostate tumor, wherein,
a stable structure corresponding to a prostate-tumor-related substance is used as the substance for treating and/or preventing the prostate tumor;
the stable structure corresponding to the substance is a structure of the substance in solid form under high temperature over 500°C.

26. The method according to claim 25, wherein, the stable structure corresponding to the prostate-tumor-related substance is selected from one or more stable structure of the group consisting of:
(1) a stable structure corresponding to a substance that causes a prostate tumor lesion in an organism or the corresponding substance thereof that does not cause a prostate tumor lesion;
(2) a stable structure corresponding to the cells, tissues or organs where the prostate tumor occurs;
(3) a stable structure corresponding to the healthy cell, tissue or organ corresponding to the primary site of the prostate tumor;
(4) a stable structure corresponding to a substance containing the substance in any of the above (1) to (3).

27. A substance for treating and/or preventing the prostate tumor designed according to the method of claims 25 or 26.

28. A method of preparing the substance for treating and/or preventing a prostate tumor according to claim 27, wherein,
the stable structure corresponding to the substance is prepared by high-temperature carbonization.

29. A method of designing a substance for treating and/or preventing a mesenchymal tissue tumor, wherein,
a stable structure corresponding to a mesenchymal-tissue-tumor-related substance is used as the substance for treating and/or preventing the mesenchymal tissue tumor;
the stable structure corresponding to the substance is a structure of the substance in solid form under high temperature over 500°C.

30. The method according to claim 29, wherein, the stable structure corresponding to the mesenchymal-tissue-tumor-related substance is selected from one or more stable structure of the group consisting of:
(1) a stable structure corresponding to a substance that causes a mesenchymal tissue tumor lesion in an organism or the corresponding substance thereof that does not cause a mesenchymal tissue tumor lesion;
(2) a stable structure corresponding to the cells, tissues or organs where the mesenchymal tissue tumor occurs;
(3) a stable structure corresponding to the healthy cell, tissue or organ corresponding to the primary site of the mesenchymal tissue tumor;
(4) a stable structure corresponding to a substance containing the substance in any of the above (1) to (3).

31. A substance for treating and/or preventing the mesenchymal tissue tumor designed according to the method of claims 29 or 30.

32. A method of preparing the substance for treating and/or preventing an mesenchymal tissue tumor according to claim 31, wherein,
the stable structure corresponding to the substance is prepared by high-temperature carbonization.

33. A method of designing a substance for treating and/or preventing a neuroectodermal tumor, wherein,
a stable structure corresponding to a neuroectodermal-tumor-related substance is used as the substance for treating and/or preventing the neuroectodermal tumor;
the stable structure corresponding to the substance is a structure of the substance in solid form under high temperature over 500°C.

34. The method according to claim 33, wherein, the stable structure corresponding to the neuroectodermal-tumor-related substance is selected from one or more stable structure of the group consisting of:
a stable structure corresponding to a substance that causes a neuroectodermal tumor lesion in an organism or the corresponding substance thereof that does not cause a neuroectodermal tumor lesion;
(2) a stable structure corresponding to the cells, tissues or organs where the neuroectodermal tumor occurs;
(3) a stable structure corresponding to the healthy cell, tissue or organ corresponding to the primary site of the neuroectodermal tumor;
(4) a stable structure corresponding to a substance containing the substance in any of the above (1) to (3).

35. A substance for treating and/or preventing the neuroectodermal tumor designed according to the method of claims 33 or 34.

36. A method of preparing the substance for treating and/or preventing a neuroectodermal tumor according to claim 35, wherein,
the stable structure corresponding to the substance is prepared by high-temperature carbonization.

37. A method of designing a substance for treating and/or preventing leukemia, wherein,
a stable structure corresponding to a leukemia-related substance is used as the substance for treating and/or preventing the leukemia;
the stable structure corresponding to the substance is a structure of the substance in solid form under high temperature over 500°C .

38. The method according to claim 37, wherein, the stable structure corresponding to the leukemia-related substance is selected from one or more stable structure of the group consisting of:
(1) a stable structure corresponding to a substance that causes leukemia lesion in an organism or the corresponding substance thereof that does not cause leukemia lesion;
(2) a stable structure corresponding to the cells, tissues or organs where leukemia occurs;
(3) a stable structure corresponding to the healthy cell, tissue or organ corresponding to the primary site of the leukemia;
(4) a stable structure corresponding to a substance containing the substance in any of the above (1) to (3).

39. A substance for treating and/or preventing the leukemia designed according to the method of claims 37 or 38.

40. A method of preparing the substance for treating and/or preventing leukemia according to claim 39, wherein,
the stable structure corresponding to the substance is prepared by high-temperature carbonization.

41. Use of the substance according to any one of claims 3, 7, 11, 15, 19, 23, 27, 31, 35, 39 in the manufacture of a medicament, health product, food, or food additive.
